# EUROPEAN PATENT SPECIFICATION

(11) **EP 4 131 283 B1**
(45) Date of publication and mention of the grant of the patent: **10.09.2025**
(21) Application number: 22168515.9
(22) Date of filing: 14.04.2022
(51) Int. Cl.: G16H 50/20, A61B 5/00

(54) **CARDIAC EVENT IDENTIFIER AND SELECTOR**
IDENTIFIZIERER UND AUSWAHLVORRICHTUNG FÜR HERZEREIGNISSE
IDENTIFICATEUR ET SÉLECTEUR D'ÉVÉNEMENT CARDIAQUE

(30) Priority: 15.04.2021 US 202117232036
(43) Date of publication of application: 08.02.2023
(73) Proprietor: Boston Scientific Cardiac Diagnostics, Inc., Rochester MN 55901 (US)
(72) Inventor: SMITH, Richard M., Rochester, 55901 (US); MCROBERTS, Michael, Rochester, 55901 (US); SIMON, Eric, Rochester, 55901 (US)
(74) Representative: Pfenning, Meinig & Partner mbB

(56) References cited:
- US-A1- 2020 155 025
- US-B2- 10 470 702

## Description

### BACKGROUND

Portable monitoring devices for collecting biometric data are becoming increasingly common in diagnosing and treating medical conditions in patients. For example, mobile devices can be used to monitor cardiac data in a patient. This cardiac monitoring can empower physicians with valuable information regarding the occurrence and regularity of a variety of heart conditions and irregularities in patients. Cardiac monitoring can be used, for example, to identify abnormal cardiac rhythms, so that critical alerts can be provided to patients, physicians, or other care providers and patients can be treated.

US 2020/155025 A1 discusses techniques for identifying onset of a cardiac event. Embodiments receive biometric data measured by at least one remote monitoring device, the biometric data comprising an electrocardiogram (ECG) data relating to a patient. It is determined that the ECG data includes a plurality of critical rhythms, and one of the plurality of critical rhythms is identified as an onset event. The ECG data is modified, based on the identified onset event. The modified ECG data facilitates treatment of the patient related to the identified onset event.

### SUMMARY

This application relates to a method for identifying and selecting cardiac events. The method comprises a step of retrieving a plurality of indications of cardiac events in a patient; heart rates of the patient during the cardiac events; and durations of the cardiac events.

Afterwards the method comprises a step of selecting a subset of indications from the plurality of indications based on the heart rates of the patient during the cardiac events and the durations of the cardiac events; wherein selecting the subset of indications comprises eliminating a first indication of the plurality of indications in response to determining that (i) the first indication indicates a same type of cardiac event as a second indication of the plurality of indications and (ii) a difference between a heart rate indicated by the first indication and a heart rate indicated by the second indication is less than a difference threshold.

Then, the method comprises a step of sorting the subset of indications based on heart rates of the patient during the cardiac event indicated by the subset of indications and based on durations of the cardiac events indicated by the subset of indications to produce a sorted list of indications.

The method further comprises a step of communicating, from the sorted list, an indication that is first in the sorted list for clinical review.

Optionally, in the described method, the plurality of indications are generated based on electrocardiogram measurements of the patient, wherein the plurality of indications further indicate confidence levels of the electrocardiogram measurements, and wherein selecting the subset of indications is further based on the confidence levels of the electrocardiogram measurements.

Optionally, in the described method, sorting the subset of indications is further based on the confidence levels indicated by the subset of indications.

Optionally, in the described method the plurality of indications further indicate beat counts of the patient during the cardiac events and wherein selecting the subset of indications is further based on the beat counts.

Optionally, in the described method, eliminating the first indication is based on a duration indicated by the first indication being shorter than a duration indicated by the second indication.

Optionally, in the described method, the cardiac events in the patient occurred in a same day.

Optionally, in the described method, communicating the indication comprises generating a report that includes the indication.

Optionally, the described method further comprises communicating a plurality of indications from the sorted list for clinical review based on an event threshold. The application further relates to an apparatus comprising a memory; and a hardware processor communicatively coupled to the memory, the hardware processor configured to retrieve a plurality of indications of cardiac events in a patient; heart rates of the patient during the cardiac events; and durations of the cardiac events.

The processor is further configured to select a subset of indications from the plurality of indications based on the heart rates of the patient during the cardiac events and the durations of the cardiac events; wherein selecting the subset of indications comprises eliminating a first indication of the plurality of indications in response to determining that (i) the first indication indicates a same type of cardiac event as a second indication of the plurality of indications and (ii) a difference between a heart rate indicated by the first indication and a heart rate indicated by the second indication is less than a difference threshold.

The processor is further configured to sort the subset of indications based on heart rates of the patient during the cardiac event indicated by the subset of indications and based on durations of the cardiac events indicated by the subset of indications to produce a sorted list of indications; and the processor is further configured to communicate, from the sorted list, an indication that is first in the sorted list for clinical review.

Optionally, in the described apparatus, the plurality of indications are generated based on electrocardiogram measurements of the patient, wherein the plurality of indications further indicate confidence levels of the electrocardiogram measurements, and wherein selecting the subset of indications is further based on the confidence levels of the electrocardiogram measurements.

Optionally, the processor is configured to sort the subset of indications based on the confidence levels indicated by the subset of indications.

Optionally, in the described apparatus, the plurality of indications further indicate beat counts of the patient during the cardiac events and wherein selecting the subset of indications is further based on the beat counts.

Optionally, the processor is configured to eliminate the first indication based on a duration indicated by the first indication being shorter than a duration indicated by the second indication.

Optionally, in the described apparatus, the cardiac events in the patient occurred in a same day.

Optionally, the processor is configured to communicate the indication by generating a report that includes the indication.

### BRIEF DESCRIPTION OF THE DRAWINGS

So that the manner in which the features of the present disclosure can be understood in detail, a more particular description of the disclosure, briefly summarized above, may be had by reference to examples, some of which are illustrated in the appended drawings.
Figure 1 illustrates an example system.
Figure 2 is a flowchart of an example method in the system of Figure 1.
Figure 3 is a flowchart of an example method in the system of Figure 1.
Figure 4 illustrates an example cardiac event server in the system of Figure 1.

To facilitate understanding, identical reference numerals have been used, where possible, to designate identical elements that are common to the figures. It is contemplated that elements and features of one example may be beneficially incorporated in other example without further recitation.

### DESCRIPTION OF EXAMPLES

### EXAMPLES

As part of mobile cardiac monitoring, a remote monitoring device can collect electrocardiogram (ECG) data for a patient, and transmit the ECG data to a remote server or device for classification and processing. This ECG data can be transmitted in strips, representing ECG readings for a particular period of time (e.g., a few seconds, a few minutes, or longer). A remote server or device can evaluate the strip of ECG data to identify abnormal cardiac rhythms, or other issues, and generate events.

In some circumstances, multiple abnormal cardiac rhythms or other issues can be seen in a given strip ECG of data for a patient, or across multiple ECG data strips for the patient. These abnormal cardiac rhythms (or other issues) can signify cardiac events for a patient. A remote server or device evaluating the ECG data can identify the cardiac events and notify a lab for clinical review. Appropriate care may then be administered to the patient based on the clinical review.

Many patients, however, experience numerous cardiac events over a short period of time. When these events are all reported to the lab, the lab may become inundated with data to review. Additionally, much of the data may not result in the patient receiving different or better care than what the patient was already receiving. For example, if a patient experiences the same type of cardiac event of a similar magnitude several times during a day, all of these events need not be reported to the lab for appropriate care to be determined. When all of these events are sent for clinical review, the lab becomes tasked with unnecessary reviews, which jeopardizes the health of other patients as review of their data is delayed.

This disclosure describes a server that uses machine learning to identify the cardiac events detected in a patient and to determine whether those events should be communicated for further clinical review. The server filters the cardiac events of a patient using thresholds for different characteristics of the cardiac event (e.g., heart rates, durations, beat counts, etc.) to reduce the number of cardiac events for analysis. After the cardiac events are filtered, the server sorts the remaining events based on one or more factors (e.g., heart rates, durations, and/or confidence levels of the classified cardiac events). The server then communicates one or more of the events from the sorted list for clinical review. For example, the server may communicate a certain number of events from the top of the sorted list. In this manner, the amount of data communicated and reviewed is reduced, which improves the health and care of other patients in particular examples.

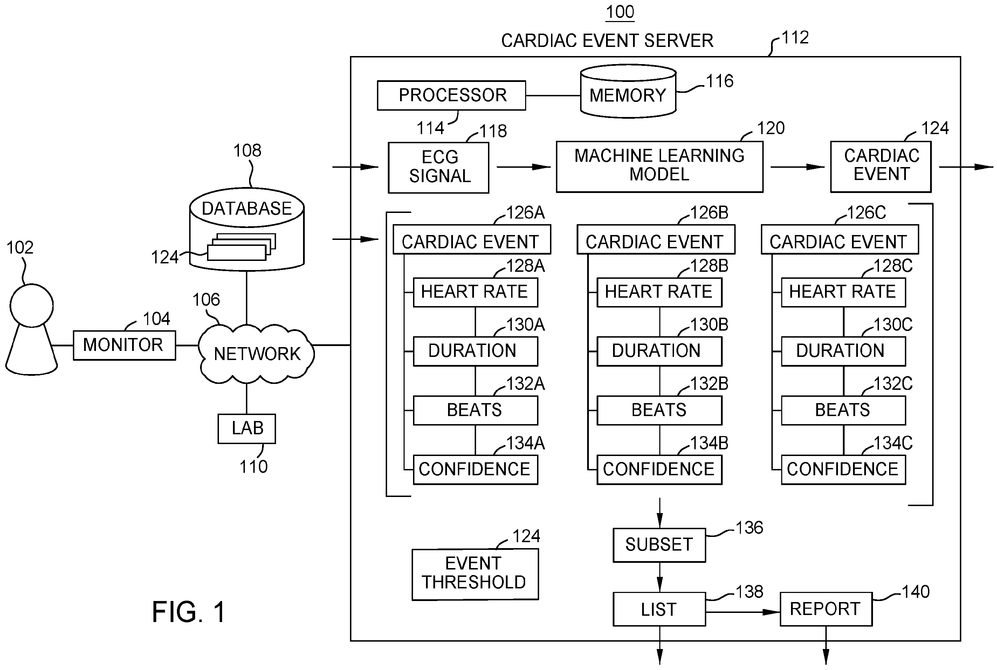

Figure 1 illustrates an example system 100. As seen in Figure 1, the system 100 includes one or more monitors 104, a network 106, a database 108, a lab 110, and a cardiac event server 112. The cardiac event server 112 identifies cardiac events in the patient 102 based on ECG signals provided by the monitor 104. The cardiac event server 112 reduces the number of cardiac events reported to the lab 110 for clinical review by filtering the cardiac events of the patient 102 based on characteristics of the cardiac events. After the cardiac events are filtered, the cardiac event server 112 sorts the remaining cardiac events to form a sorted list of cardiac events. The cardiac event server 112 then communicates a number of cardiac events from the top of the sorted list to the lab 110 for clinical review. In this manner, the amount of information that the lab 110 reviews is reduced, which improves the health and care of the patient 102 and other patients 102, in particular examples.

The monitor 104 is a device that couples to a patient 102 to detect cardiac activity of the patient 102. The monitor 104 may attach to the patient 102 using any suitable mechanism. For example, the monitor 104 may include an adhesive that couples the monitor 104 to the body of the patient 102. As another example, the monitor 104 may include a clip that couples the monitor 104 to a casing attached to the patient 102. After the monitor 104 is attached to the patient 102, the monitor 104 may detect cardiac activity within the patient 102. The monitor 104 may produce electric signals that represent the cardiac activity in the patient 102. For example, the monitor 102 may detect the patient's 102 heart beating (e.g., using infrared sensors, electrodes, etc.) and convert the detected heartbeat into electric signals. The monitor 104 communicates the electric signals to the cardiac event server 112 for analysis. For example, the cardiac event server 112 may classify the electric signals as representing regular or irregular cardiac activity. Additionally, the cardiac event server 112 may further classify irregular cardiac activity as particular cardiac events.

The network 106 is any suitable network operable to facilitate communication between the components of the system 100. The network 106 may include any interconnecting system capable of transmitting audio, video, signals, data, messages, or any combination of the preceding. The network 106 may include all or a portion of a public switched telephone network (PSTN), a public or private data network, a local area network (LAN), a metropolitan area network (MAN), a wide area network (WAN), a local, regional, or global communication or computer network, such as the Internet, a wireline or wireless network, an enterprise intranet, or any other suitable communication link, including combinations thereof, operable to facilitate communication between the components.

The database 108 stores cardiac events of the patient 102. The cardiac event server 112 identifies cardiac events of the patient 102, based on ECG signals received from the monitor 104. After the cardiac event server 112 identifies the cardiac events, the cardiac event server 112 stores the cardiac events in the database 108 for subsequent retrieval and analysis. The cardiac event server 112 may store any characteristic of the cardiac event along with the cardiac event in the database 108. For example, the cardiac event server 112 may store heart rates, durations, beat counts, and confidence levels associated with the cardiac events in the database 108. The cardiac event server 112 may subsequently retrieve the cardiac events and their associated characteristics from the database 108 for analysis.

The lab 110 includes equipment and clinicians that review information from the cardiac event server 112 to determine the appropriate care to administer to a patient 102. For example, the lab 110 performs clinical review of cardiac events communicated by the cardiac event server 112. Based on the clinical review of the cardiac events, the lab 110 determines the best care to administer to a patient 102 to preserve or improve the health of the patient 102. If too much information from too many patients 102 is communicated to the lab 110, then the lab 110 may get backed up and review of the information for certain patients 102 may be delayed. As a result, the health of these patients 102 may be jeopardized.

The cardiac event server 112 identifies cardiac events of a patient 102 based on ECG signals received from the monitor 104. After the cardiac events are identified, the cardiac event server 112 filters the cardiac events to reduce the number of cardiac events for analysis. The cardiac event server 112 then sorts the remaining cardiac events to produce a sorted list of cardiac events. The cardiac event server 112 communicates a number of cardiac events from the top of the sorted list to the lab 110 for clinical review. In this manner, the cardiac event server 112 reduces the number of cardiac events communicated to the lab 110 for clinical review, which improves the heath and care of the patient 102 and of other patients 102, in particular examples. As seen in Figure 1, the cardiac event server 112 includes a processor 114 and a memory 116, which are configured to perform any of the functions or actions of the cardiac event server 112 described herein.

The processor 114 is any electronic circuitry, including, but not limited to microprocessors, application specific integrated circuits (ASIC), application specific instruction set processor (ASIP), and/or state machines, that communicatively couples to memory 116 and controls the operation of the cardiac event server 110. The processor 114 may be 8-bit, 16-bit, 32-bit, 64-bit or of any other suitable architecture. The processor 114 may include an arithmetic logic unit (ALU) for performing arithmetic and logic operations, processor registers that supply operands to the ALU and store the results of ALU operations, and a control unit that fetches instructions from memory and executes them by directing the coordinated operations of the ALU, registers and other components. The processor 114 may include other hardware that operates software to control and process information. The processor 114 executes software stored on the memory 116 to perform any of the functions described herein. The processor 114 controls the operation and administration of the cardiac event server 110 by processing information (e.g., information received from the monitors 104, network 106, and memory 116). The processor 114 may be a programmable logic device, a microcontroller, a microprocessor, any suitable processing device, or any suitable combination of the preceding. The processor 114 is not limited to a single processing device and may encompass multiple processing devices.

The memory 116 may store, either permanently or temporarily, data, operational software, or other information for the processor 114. The memory 116 may include any one or a combination of volatile or non-volatile local or remote devices suitable for storing information. For example, the memory 116 may include random access memory (RAM), read only memory (ROM), magnetic storage devices, optical storage devices, or any other suitable information storage device or a combination of these devices. The software represents any suitable set of instructions, logic, or code embodied in a computer-readable storage medium. For example, the software may be embodied in the memory 116, a disk, a CD, or a flash drive. In particular examples, the software may include an application executable by the processor 114 to perform one or more of the functions described herein.

The cardiac event server 112 receives ECG signals 118 from the monitor 104. The ECG signal 118 represents the cardiac activity of the patient 102. The cardiac event server 110 analyzes the ECG signal 118 to identify and classify the cardiac activity of the patient 102. For example, the cardiac event server 110 may determine whether the cardiac activity is regular or irregular. Additionally, if the cardiac activity is irregular, the cardiac event server 110 may identify or classify a particular cardiac event that is occurring in the patient 102.

The cardiac event server 112 applies a machine learning model 120 (e.g., a deep neural network) to the ECG signal 118 to classify the cardiac activity of the patient 102. For example, the machine learning model 118 may compare the ECG signal 118 to labeled ECG signals to determine which labeled ECG signal the ECG signal 118 most closely resembles. Some of the labeled ECG signals may identify a particular cardiac event. If the cardiac event server 112 or the machine learning model 120 determines that the ECG signal 118 most closely resembles a labeled ECG signal associated with a cardiac event, then the cardiac event server 112 or the machine learning model 120 may determine that the patient 102 is experiencing that cardiac event 124. Additionally, the machine learning model 120 may measure or determine certain characteristics of the cardiac activity of the patient 102 based on the ECG signal 118. For example, the cardiac event server 112 or the machine learning model 120 may determine a heart rate, a duration, or a beat count of the patient 102 during the cardiac event based on the ECG signal 118.

The machine learning model 120 may also determine a confidence level for each classification or identification of a cardiac event 124. The confidence level is an indication of certainty or uncertainty in the accuracy of the machine learning model's 120 classification or identification. For example, if the machine learning model 120 determines that there are multiple labeled ECG signals that closely resemble the ECG signal 118, then the machine learning model 120 may assign a low confidence level in the machine learning model's 120 identification or classification for that ECG signal 118. As another example, if the machine learning model 120 determines that there is only one labeled ECG signal that closely resembles the ECG signal 118, then the machine learning model 120 may assign a high level of confidence in the machine learning model's 120 identification or classification for that ECG signal 118.

After the machine learning model 120 identifies a cardiac event 124 based on the ECG signal 118, the cardiac event server 112 stores the cardiac event 124 in the database 108. The cardiac event server 112 communicates the cardiac event 124 to the database 108 for storage. Subsequently, the cardiac event server 112 may retrieve the cardiac event 124 from the database 108 for analysis. In certain examples, the cardiac event server 112 or the machine learning model 120 measures or determines certain characteristics of the cardiac event 124. For example, the cardiac event server 112 or the machine learning model 120 determines the heart rate of the patient 102 during the cardiac event 124, a duration of the cardiac event 124, a beat count of the patient 102 during the cardiac event 124, or a confidence level in the machine learning models 120 identification of the cardiac event 124. The cardiac event server 112 stores these characteristics along with the cardiac event 124 in the database 108.

The cardiac event server 112 retrieves one or more cardiac events 126 from the database 108 for analysis. For example, the cardiac event server 112 may retrieve one or more cardiac events 126 from the database 108 periodically. As another example, the cardia event server 112 may retrieve one or more cardiac events 126 from the database 108 when the cardiac event server 112 identifies a cardiac event occurring in the patient 102. In the example of Figure 1, the cardiac event server 112 retrieves cardiac events 126A, 126B, and 126C. Each of these cardiac events 126 may have previously occurred in the patient 102 and may have been previously identified by the machine learning model 120. Each of the cardiac events 126A, 126B and 126C may have occurred in the patient 102 over a span of time, such as for example one day. The cardiac event server 112 analyzes these cardiac events 126 and their corresponding characteristics to determine a number of the cardiac events 126 to communicate to the lab 110 for clinical review.

In some examples, the cardiac event server 112 retrieves the cardiac events 126 that occurred over a span of time (e.g., a day). In certain examples, the cardiac event server 112 selects different spans of time for which cardiac events 126 should be retrieved. For example, the cardiac event server 112 may select the thirty-five days over a forty-five day span in which the most number of cardiac events 126 were recorded. The cardiac event server 112 then retrieves the cardiac events 126 recorded during those thirty-five days.

The cardiac event server 112 also retrieves characteristics corresponding to these cardiac events 126. In the example of Figure 1, the cardiac event server 112 also retrieves one or more of a heart rate 128 of the patient 102 during the cardiac event 126, a duration of the cardiac event 126, a beat count 132 of the cardiac event 126, and a confidence level 134 of the machine learning model's 120 identification of the cardiac event 126. As seen in Figure 1, the cardiac event 126A is associated with a heart rate 128A, a duration 130A, a beat count 132A, and a confidence level 134A. The cardiac event 126B is associated with a heart rate 128B, a duration 130B, a beat count 132B, and a confidence level 134B. The cardiac event 126C is associated with a heart rate 128C, a duration 130C, a beat count 132C, and a confidence level 134C. The cardiac event server 112 analyzes these characteristics of the cardiac events 126 to reduce the number of cardiac events 126 that are reported to the lab 110 for clinical review.

The cardiac event server 112 first filters the cardiac events 126 based on the characteristics to form a subset 136 of the cardiac events 126. The cardiac event server 112 may apply thresholds to the various characteristics to determine a subset 136. For example, the cardiac event server 112 may apply minimum and maximum heart rate thresholds to the heart rates 128 of the cardiac events 126. Cardiac events 126 with heart rates 128 that fall outside of these minimum and maximum thresholds are eliminated from the subset 136. As another example, the cardiac event server 112 may apply a minimum duration threshold to the durations 130 of the cardiac events 126. The cardiac events 126 with durations 130 that fall below the duration threshold are eliminated from the subset 136. As yet another example, the cardiac event server 112 may apply a minimum beat threshold to the beat counts 132 of the cardiac events 126. The cardiac events 126 with beat counts 132 that fall below the beat threshold are eliminated from the subset 136. As yet another example, the cardiac event server 112 applies a minimum confidence threshold to the confidence levels 134 of the cardiac events 126. The cardiac events 126 with confidence levels 134 that fall below the confidence threshold are eliminated from the subset 136. In this manner, the cardiac event server 112 filters out the cardiac events 126 associated with clinically insignificant heart rates 128, durations 130, or beat counts 132. Additionally, the cardiac event server 112 filters out the cardiac events 126 for which the machine learning model's 120 identification is uncertain. In this manner, the subset 136 includes cardiac events 126 that are clinically significant and have confidence levels 134 that indicate that the machine learning model's 120 identification is accurate.

In certain examples, the cardiac event server 112 eliminates certain cardiac events 126 from the subset 136, based on a similarity of the cardiac event 126 with another cardiac event 126. Using the example of Figure 1, the cardiac event server 112 may determine that the cardiac event 126A and the cardiac event 126B are similar. The cardiac event server 112 may determine that the cardiac event 126A and the cardiac event 126B are of the same type. The cardiac event server 112 may then determine that the heart rate 128A and the heart rate 128B are similar, such that a difference between the heart rate 128A and the heart rate 128B falls below a difference threshold. Additionally or alternatively, the cardiac event server 112 may determine that a difference between the duration 130A and the duration 130B or a difference between the beat count 132A and the beat count 132B is below a difference in threshold. In response to these determinations, the cardiac event server 112 may eliminate one of the cardiac event 126A or the cardiac event 126B from the subset 136. For example, the cardiac event server 112 may select the cardiac event 126A or 126B with the higher heart rate 128A or 128B to be included in the subset 136. As another example, the cardiac event server 112 may select the cardiac event 126A or 126B with the longer duration 130A or 130B. In this manner, the cardiac event server 112 eliminates cardiac events 126 that are clinically redundant, which reduces the number of cardiac events 126 to be reported to the lab 110 for clinical review.

After forming the subset 136, the cardiac event server 112 sorts the subset 136 based on one or more criterion to form a sorted list 138. For example, the cardiac event server 112 may sort the cardiac events 126 in the subset 136 based on their heart rates 128, durations 130, and/or confidence levels 134. The cardiac event server 112 may sort the subset 136 such that the cardiac events 126 at the top of the sorted list 138 are the most severe cardiac events 126. For example, a cardiac event 126 with the highest heart rate 128, the longest duration 130, and the highest confidence level 134 may be at the top of the sorted list 138. A cardiac event 126 with the lowest heart rate 128, the lowest duration 130, and/or the lowest confidence level 134 may be near the bottom of the sorted list 138. As a result, the sorted list 138 indicates the most severe cardiac events 126 that occurred in the patient 102. The cardiac event server 112 then communicates a certain number of cardiac events 126 from the top of the sorted list 138 to the lab 110 for clinical review. In this manner, the lab 110 reviews only the most clinically significant and severe cardiac events 126 that occurred in the patient 102. As a result, the amount of information that the lab 110 is asked to review is reduced, which improves the health or care of the patient 102 and other patients 102 in particular examples.

In certain examples, the cardiac event server 112 generates a report 130 based on the sorted list 138. For example, the report 140 may include a number of cardiac events 126 from the top of the sorted list 138. The report 140 may include the heart rates 128, durations 130, and/or beat counts 132 of these cardiac events 126. The cardiac event server 112 may communicate the report 140 to a clinician for further review. Moreover, the cardiac event server 112 may communicate the report 140 to the lab 110 for clinical review. In this manner, the report 140 provides information on the most clinically significant and severe cardiac events 126 that occurred in the patient 102.

In some examples, the cardiac event server 112 communicates cardiac events 126 to the lab 110 for clinical review based on an event threshold 142. The event threshold 142 may limit the number of cardiac events 126 to be communicated to the lab 110 over a span of time (e.g., a day). The cardiac event server 112 may select a number of cardiac events 126 from the top of the sorted list 138 based on the event threshold 142. For example, if the event threshold 142 is three events, then the cardiac event server 112 may communicate the top three events from the sorted list 138 to the lab 110 per day. In this manner, the event threshold 142 reduces the amount of information communicated to the lab 110 for clinical review.

The cardiac event server 112 may implement multiple event thresholds 142 for different types of cardiac events 126. These event thresholds 142 limit the number of cardiac events 126 of particular types that are communicated over a span of time. For example, an event threshold 142 may limit the number of cardiac events 126 of a particular type that is communicated to the lab 110 in the morning or in the evening. As another example, an event threshold 142 may limit the number of cardiac events 126 of a particular type that are communicated to the lab 110 per day. As yet another example, an event threshold 142 may limit the number of cardiac events 126 of a first type that are communicated to the lab 110 if a cardiac event 126 of another type is being communicated to the lab 110. In some instances, the event threshold 142 for a particular type of cardiac event 126 may set a minimum number of cardiac events 126 of the particular type to be communicated to the lab 110 over a span of time (e.g., at least one cardiac event 126 of that type must be communicated per day).

In some examples, the cardiac event server 112 adjusts the event threshold 142 and/or the filtering thresholds based on one or more factors. For example, the cardiac event server 112 may adjust the event threshold 142 and/or the filtering thresholds based on the health of a patient 102, the age of the patient 102, the time of day when the cardiac event 126 occurred.

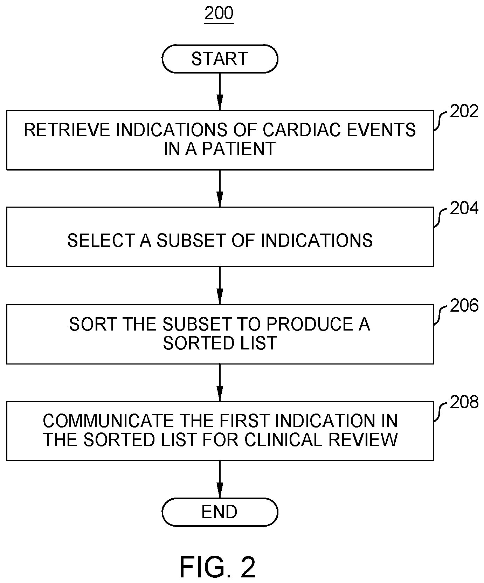

Figure 2 is a flowchart of an example method 200 in the system 100 of Figure 1. The cardiac event server 112 performs the method 200. In particular examples, by performing the method 200, the cardiac event server 112 reduces the number of cardiac events of a patient 102 that are communicated to a lab 110 for clinical review, which improves the health and care of the patient 102 and other patients 102.

In block 202, the cardiac event server 112 retrieves indications of cardiac events 126 in a patient 102 from a database 108. These cardiac events 126 may have been previously identified by the cardiac event server 112 based on ECG signals 118 provided by a monitor 104 attached to the patient 102. The cardiac event server 112 may also retrieve characteristics of these cardiac events 126 from the database 108. For example, the cardiac event server 112 may retrieve heart rates 128 of the patient 102 during the cardiac events 126, durations 130 of the cardiac events 126, beat counts 132 during the cardiac event 126, and confidence levels 134 of the machine learning model's 120 identification of the cardiac events 126. The cardiac event server 112 analyzes the cardiac events 126 along with their characteristics to determine which cardiac events 126 should be communicated to the lab 110 for clinical review.

In block 204, the cardiac event server 112 selects a subset 136 of indications of cardiac events 126. The cardiac event server 112 may select the subset 136 based on the characteristics of the cardiac events 126. For example, the cardiac event server 110 may apply minimum or maximum thresholds on each of the characteristics of the cardiac events 126 to select the subset 136. The cardiac event server 112 may apply a minimum and/or a maximum heart rate threshold to the heart rates 128 of the cardiac events 126. The cardiac events 126 with heart rates 128 that fall within the minimum and maximum heart rate thresholds may be selected for inclusion in the subset 136. As another example, the cardiac event 126 may apply a minimum duration threshold to the durations 130 of the cardiac events 126. The cardiac events 126 with durations 130 that exceed the minimum duration threshold are selected for inclusion in the subset 136. As yet another example, the cardiac event server 112 may apply a minimum beat threshold to the beat counts 132 of the cardiac events 126. The cardiac events 126 with beat counts 132 that exceed the minimum beat threshold are selected for inclusion in the subset 136. As yet another example, the cardiac event server 112 may apply a confidence level threshold to the cardiac events 126. The cardiac events 126 with confidence levels 134 that exceed the minimum confidence level are selected for inclusion in the subset 136. In this manner, the subset 136 includes cardiac events 126 that are clinically significant and are likely accurately identified by the machine learning model 120.

In block 206, the cardiac event server 112 sorts the subset 136 to produce a sorted list 138. The cardiac event server 112 may sort the cardiac events 126 in the subset 136 according to any number of criteria. For example, the cardiac event server 112 may sort the cardiac events 126 in the subset 136 based on their heart rates 128, durations 130, beat counts 132, and/or confidence levels 138. The cardiac event server 112 may sort the cardiac events 126 based on these characteristics to order the cardiac events 126 by severity. For example, the cardiac event server 112 may place at the top of the sorted list 138 a cardiac event 126 with the highest heart rate 128 and the longest duration 130. As another example, the cardiac event server 112 may place near the bottom of the sorted list 138 a cardiac event 126 with a lowest heart rate 128 and the lowest duration 130. In this manner, the sorted list 138 organizes the cardiac events 126 and the subset 136 based on the severity.

In block 208, the cardiac event server 112 communicates a first indication of a cardiac event 126 in the sorted list 138 to a lab 110 for clinical review. The first cardiac event 126 in the sorted list 138 may be considered the most clinically significant or severe cardiac event 126 in the patient 102. In certain examples, the cardiac event server 112 may also communicate additional cardiac events from the sorted list 138 to the lab 110 for clinical review (e.g., a second highest and third highest cardiac event 126 from the sorted list 138). The cardiac event server 112 may communicate a number of cardiac events 126 from the sorted list 138 based on an event threshold 142. The event threshold 142 may limit the number of cardiac events 126 communicated from the sorted list 138.

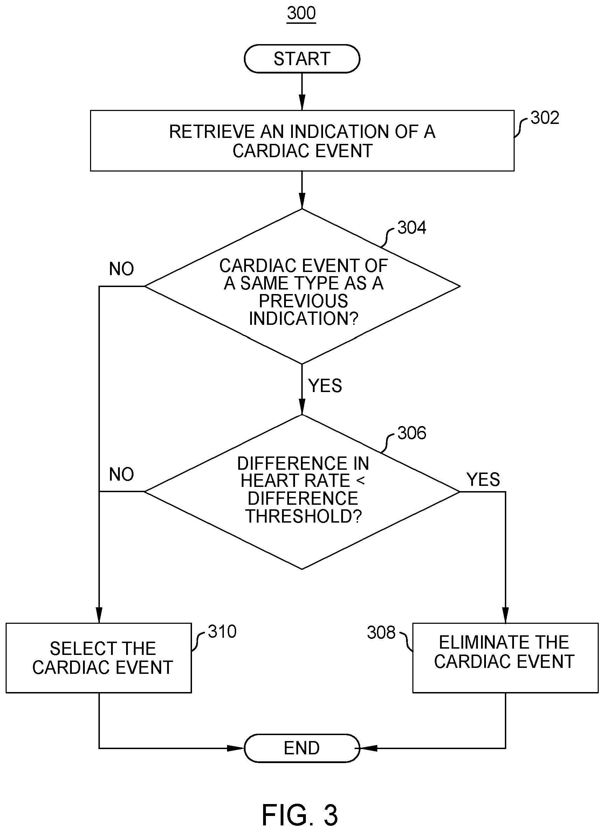

Figure 3 is a flowchart of an example method 300 in the system 100 of Figure 1. The cardiac event server 112 may perform the method 300. In particular examples, by performing the method 300, the cardiac server 112 reduces the number of cardiac events 126 to be included in the subset 136.

In block 302, the cardiac event server 112 retrieves an indication of a cardiac event 126 from the database 108. The cardiac event server 112 may have previously identified the cardiac event 126 by applying a machine learning model 120 to the ECG signals 118 received from a monitor 104. The cardiac event server 112 may also retrieve characteristics of the cardiac event 126. For example, the cardiac event server 112 may retrieve a heart rate 128, a duration 130, a beat count 132, and/or a confidence level 134 of the cardiac event 126 from the database 108. The cardiac event server 112 may then analyze the cardiac event 126 relative to other cardiac events 126 retrieved from the database 108 to eliminate similar cardiac events 126 from the subset 136.

In block 304, the cardiac event server 112 determines whether the cardiac event 126 is of a same type as a previous indication of a cardiac event 126 retrieved from the database 108. If the cardiac event 126 does not have the same type as previous cardiac events 126, then the cardiac event server 112 considers the cardiac event 126 as the first occurrence of the cardiac event 126 in the patient 102. In response, the cardiac event server 112 selects the cardiac event 126 for inclusion in the subset 136 in block 310.

If the cardiac event 126 is of the same type as another cardiac event 126, then the cardiac event server 112 determines whether a difference in heart rates 128 between the two cardiac events 126 falls below a difference threshold. If the difference in heart rates 128 falls below the difference threshold, then the cardiac event server 112 considers the two cardiac events 126 as similar. In response, the cardiac event server 112 eliminates the cardiac event 126 from inclusion in the subset 136. If the cardiac event server 112 determines that the differences in heart rates 128 exceeds the difference threshold, then the cardiac event server 112 may select the cardiac event 126 for inclusion in the subset 136 in block 301.

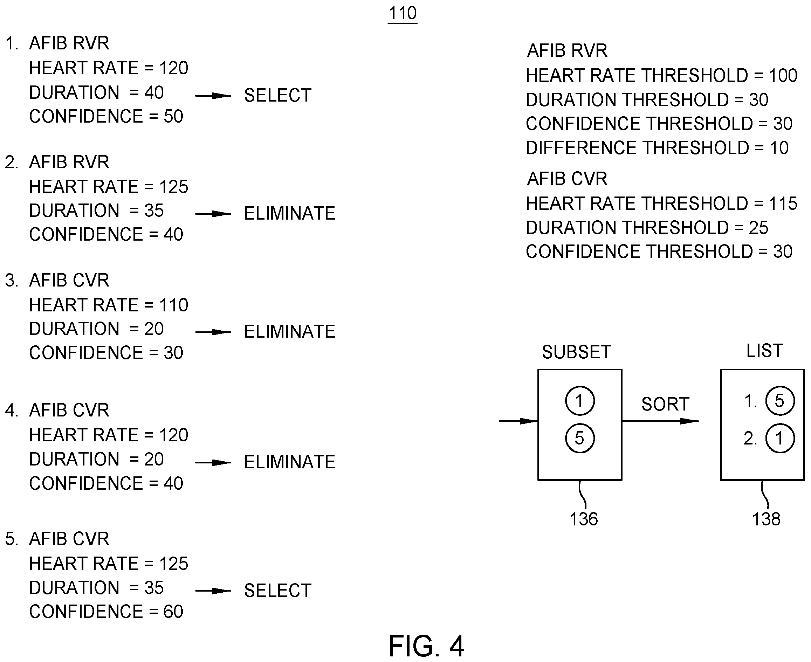

Figure 4 illustrates an example cardiac event server 112 in the system 100 of Figure 1. In the example of Figure 4, the cardiac event server 112 filters out identified cardiac events to form a subset 136 of cardiac events. The cardiac event server 112 then sorts the subset 136 to produce a sorted list 138 of cardiac events.

The cardiac event server 112 retrieves five cardiac events 126 from the database 108. The cardiac event server 112 retrieves a first cardiac event, which is identified as an atrial fibrillation with rapid ventricular response (AFib RVR). A patient 102 also had a heart rate of 120 during the first instance of AFib RVR. Additionally, the duration of the first instance of AFib RVR was 40 and a confidence level of the identification of the first instance of AFib RVR is 50.

The cardiac event server 112 also retrieves a second instance of AFib RVR. A heart rate of the patient 102 during the second instance of AFib RVR was 125. The duration of the second instance of AFib RVR was 35 and a confidence level of the identification of the second instance of AFib RVR was 40.

The cardiac event server 112 also retrieves a first instance of atrial fibrillation with controlled ventricular rate (AFib CVR) from the database 108. The patient 102 had a heart rate of 110 during the first instance of AFib CVR. The first instance of AFib CVR had a duration of 20. A confidence level of the identification of the first instance of AFib CVR was 30.

The cardiac event server 112 also retrieves a second instance of AFib CVR from the database 108. The patient 102 had a heart rate of 120 during the second instance of AFib CVR. The second instance of AFib CVR had a duration of 20. A confidence level of the identification of the second instance of AFib CVR was 40.

The cardiac event server 112 retrieves a third instance of AFib CVR from the database 108. A heart rate of the patient 102 during the third instance of AFib CVR was 125. The duration of the third instance of AFib CVR was 35. A confidence level of the identification of the third instance of AFib CVR was 60.

The cardiac event server 112 filters the five retrieved cardiac events to determine the subset 136. The cardiac event server 112 may apply thresholds to various characteristics of these cardiac events to filter out the cardiac events from the subset 136. In the example of Figure 4, the cardiac event server 112 applies a heart rate threshold of 100, a duration threshold of 30, and a confidence threshold of 30 to AFib RVR events. Additionally, the cardiac event server 112 applies a heart rate threshold of 115, a duration threshold of 25, and a confidence threshold of 30 to AFib CVR events. By applying these thresholds to the five cardiac events, the cardiac event server 112 selects the first AFib RVR event and the third AFib CVR event for inclusion in the subset 136.

The cardiac event server 112 selects the first AFib RVR event because the heart rate during the first AFib RVR and the duration of the first AFib RVR event exceed the heart rate threshold and the duration threshold for AFib RVR events. Additionally, the confidence level of the identification of the first AFib RVR event exceeds the confidence threshold for AFib RVR events.

The cardiac event server 112 eliminates the second AFib RVR event because the second AFib RVR event is too similar to the first AFib RVR event. Specifically, the cardiac event server 112 determines that the difference between the heart rates and durations of the first and second AFib RVR events do not exceed a difference threshold of 10 for AFib RVR events. In response, the cardiac event server 112 eliminates the second AFib RVR event from inclusion in the subset 136.

The cardiac event server 112 eliminates the first AFib CVR event because the heart rate and duration of the first AFib CVR event do not exceed the heart rate threshold and duration threshold for AFib CVR events. The cardiac event server 112 also eliminates the second AFib CVR event because the duration of the second AFib CVR event does not exceed the duration threshold of AFib CVR events. As a result, even though the heart rate of the second AFib CVR event exceeds the heart rate threshold of AFib CVR events the cardiac event server 112 still does not select the second AFib CVR event for inclusion in the subset 136. The cardiac event server 112 selects the third AFib CVR event to be included in the subset 136 because the heart rate and duration of the third AFib CVR event exceed the heart rate threshold and duration threshold for AFib CVR events. Additionally, the confidence level of the third AFib CVR event exceeds the confidence threshold for AFib CVR events.

The cardiac event server 112 then sorts the subset 136. The cardiac event server 112 may organize the subset 136 based on any criteria. In the example of Figure 4, the cardiac event server 112 puts the third AFib CVR event at the top of the list 138 and the first AFib RVR event at the bottom of the list 138. The cardiac event server 112 may have organized the two events based on their heart rates. The AFib CVR event may have been positioned above the AFib RVR event because the AFib CVR event had a higher heart rate than the AFib RVR event. Alternatively or additionally the cardiac event server 112 may organize the events based on their confidence levels. As a result, the AFib CVR event may have been placed above the AFib RVR event because the confidence level of the AFib CVR event is higher than the confidence level of the AFib RVR event. The cardiac event server 112 then communicates the AFib CVR event from the list 138 to a lab 110 for clinical review.

In the preceding, reference is made to examples presented in this disclosure.

As will be appreciated by one skilled in the art, the examples disclosed herein may be embodied as a system, method or computer program product. Accordingly, aspects may take the form of an entirely hardware example, an entirely software example (including firmware, resident software, micro-code, *etc.*) or an example combining software and hardware aspects that may all generally be referred to herein as a "circuit," "module" or "system." Furthermore, aspects may take the form of a computer program product embodied in one or more computer readable medium(s) having computer readable program code embodied thereon.

Any combination of one or more computer readable medium(s) may be utilized. A computer readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer readable storage medium is any tangible medium that can contain, or store a program for use by or in connection with an instruction execution system, apparatus or device.

Program code embodied on a computer readable medium may be transmitted using any appropriate medium, including but not limited to wireless, wireline, optical fiber cable, RF, *etc.,* or any suitable combination of the foregoing.

Computer program code for carrying out operations for aspects of the present disclosure may be written in any combination of one or more programming languages, including an object oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

Aspects of the present disclosure are described below with reference to flowchart illustrations and/or block diagrams of methods, apparatus (systems) and computer program products according to examples presented in this disclosure. It will be understood that each block of the flowchart illustrations and/or block diagrams, and combinations of blocks in the flowchart illustrations and/or block diagrams, can be implemented by computer program instructions. These computer program instructions may be provided to a processor of a general purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks.

These computer program instructions may also be stored in a computer readable medium that can direct a computer, other programmable data processing apparatus, or other devices to function in a particular manner, such that the instructions stored in the computer readable medium produce an article of manufacture including instructions which implement the function/act specified in the flowchart and/or block diagram block or blocks.

The computer program instructions may also be loaded onto a computer, other programmable data processing apparatus, or other devices to cause a series of operational steps to be performed on the computer, other programmable apparatus or other devices to produce a computer implemented process such that the instructions which execute on the computer or other programmable apparatus provide processes for implementing the functions/acts specified in the flowchart and/or block diagram block or blocks.

The flowchart and block diagrams in the Figures illustrate the architecture, functionality and operation of possible implementations of systems, methods and computer program products according to various examples. In this regard, each block in the flowchart or block diagrams may represent a module, segment or portion of code, which comprises one or more executable instructions for implementing the specified logical function(s). It should also be noted that, in some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts, or combinations of special purpose hardware and computer instructions.

## Claims

1. A method (200) for selecting, sorting and communicating indications of cardiac events, comprising:
Retrieving (202) a plurality of indications of:
cardiac events (126A, 126B, 126C) in a patient (102);
heart rates (128A, 128B, 128C) of the patient (102) during the cardiac events (126A, 126B, 126C); and
durations (130A, 130B, 130C) of the cardiac events;
selecting (204) a subset (136) of indications from the plurality of indications based on the heart rates of the patient during the cardiac events and the durations of the cardiac events;
sorting (206) the subset (136) of indications based on heart rates of the patient during the cardiac event indicated by the subset (136) of indications and based on durations of the cardiac events indicated by the subset (136) of indications to produce a sorted list of indications; and
communicating (208), from the sorted list, an indication that is first in the sorted list for clinical review;
wherein selecting (206, 310) the subset (136) of indications comprises eliminating (308) a first indication of the plurality of indications in response to determining (304, 306) that (i) the first indication indicates a same type of cardiac event as a second indication of the plurality of indications and (ii) a difference between a heart rate indicated by the first indication and a heart rate indicated by the second indication is less than a difference threshold.

2. The method of claim 1, wherein the plurality of indications are generated based on electrocardiogram measurements (118) of the patient (102), wherein the plurality of indications further indicate confidence levels (134A, 134B, 134C) of the electrocardiogram measurements (118), and wherein selecting (204, 310) the subset (136) of indications is further based on the confidence levels (134A, 134B, 134C) of the electrocardiogram measurements (118).

3. The method of claim 2, wherein sorting (206) the subset (136) of indications is further based on the confidence levels indicated by the subset (136) of indications.

4. The method of claim 1, wherein the plurality of indications further indicate beat counts (132A, 132B, 132C) of the patient during the cardiac events (126A, 126B, 126C) and wherein selecting (204) the subset (136) of indications is further based on the beat counts (132A, 132B, 132C).

5. The method of claim 1, wherein eliminating the first indication is based on a duration indicated by the first indication being shorter than a duration indicated by the second indication.

6. The method of claim 1, wherein the cardiac events in the patient (102) occurred in a same day.

7. The method of claim 1, wherein communicating the indication comprises generating a report that includes the indication.

8. The method of claim 1, further comprising communicating a plurality of indications from the sorted list for clinical review based on an event threshold.

9. An apparatus (112) for selecting, sorting and communicating indications of cardiac events, comprising:
a memory (116); and
a hardware processor (114) communicatively coupled to the memory, the hardware processor configured to:
retrieve (202) a plurality of indications of:
cardiac events (126A, 126B, 126C) in a patient (102);
heart rates (128A, 128B, 128C) of the patient (102) during the cardiac events; and
durations (130A, 130B, 130C) of the cardiac events;
select (204) a subset of indications from the plurality of indications based on the heart rates of the patient during the cardiac events and the durations of the cardiac events;
sort (206) the subset of indications based on heart rates of the patient during the cardiac event indicated by the subset of indications and based on durations of the cardiac events indicated by the subset of indications to produce a sorted list of indications; and
communicate (208), from the sorted list, an indication that is first in the sorted list for clinical review;
wherein selecting (204, 310) the subset of indications comprises eliminating (308) a first indication of the plurality of indications in response to determining (304, 306) that (i) the first indication indicates a same type of cardiac event as a second indication of the plurality of indications and (ii) a difference between a heart rate indicated by the first indication and a heart rate indicated by the second indication is less than a difference threshold.

10. The apparatus (112) of claim 9, wherein the plurality of indications are generated based on electrocardiogram measurements of the patient, wherein the plurality of indications further indicate confidence levels of the electrocardiogram measurements, and wherein selecting the subset (136) of indications is further based on the confidence levels of the electrocardiogram measurements.

11. The apparatus (112) of claim 10, wherein sorting (206) the subset (136) of indications is further based on the confidence levels indicated by the subset (136) of indications.

12. The apparatus (112) of claim 9, wherein the plurality of indications further indicate beat counts (132A, 132B, 132C) of the patient (102) during the cardiac events and wherein selecting (204) the subset (136) of indications is further based on the beat counts.

13. The apparatus (112) of claim 9, wherein eliminating (308) the first indication is based on a duration indicated by the first indication being shorter than a duration indicated by the second indication.

14. The apparatus (112) of claim 9, wherein the cardiac events in the patient (102) occurred in a same day.

15. The apparatus (112) of claim 9, wherein communicating the indication comprises generating a report that includes the indication.

## Patentansprüche

1. Verfahren (200) zum Auswählen, Sortieren und Übermitteln von Angaben zu kardialen Ereignissen, umfassend:
Abrufen (202) mehrerer Angaben zu:
kardialen Ereignissen (126A, 126B, 126C) bei einem Patienten (102);
Herzfrequenzen (128A, 128B, 128C) des Patienten (102) während der kardialen Ereignisse (126A, 126B, 126C); und
Dauern (130A, 130B, 130C) der kardialen Ereignisse;
Auswählen (204) einer Teilmenge (136) der Angaben aus den mehreren Angaben basierend auf den Herzfrequenzen des Patienten während der kardialen Ereignisse und den Dauern der kardialen Ereignisse;
Sortieren (206) der Teilmenge (136) der Angaben basierend auf Herzfrequenzen des Patienten während des kardialen Ereignisses, das von der Teilmenge (136) der Angaben angegeben wird, und basierend auf Dauern der kardialen Ereignisse, die von der Teilmenge (136) der Angaben angegeben werden, um eine sortierte Liste von Angaben zu erstellen; und
Übermitteln (208) einer Angabe von der sortierten Liste, die die erste auf der sortierten Liste ist, zur medizinischen Überprüfung;
wobei das Auswählen (206, 310) der Teilmenge (136) der Angaben das Eliminieren (308) einer ersten Angabe der mehreren Angaben umfasst, als Reaktion auf das Bestimmen (304, 306), dass (i) die erste Angabe einen gleichen Typ von kardialem Ereignis angibt wie eine zweite Angabe der mehreren Angaben und (ii) ein Unterschied zwischen einer von der ersten Angabe angegebenen Herzfrequenz und einer von der zweiten Angabe angegebenen Herzfrequenz geringer ist als ein Unterschiedsschwellenwert.

2. Verfahren nach Anspruch 1, wobei die mehreren Angaben basierend auf Elektrokardiogrammmessungen (118) des Patienten (102) erzeugt werden, wobei die mehreren Angaben ferner Konfidenzniveaus (134A, 134B, 134C) der Elektrokardiogrammmessungen (118) angeben und wobei das Auswählen (204, 310) der Teilmenge (136) der Angaben ferner auf den Konfidenzniveaus (134A, 134B, 134C) der Elektrokardiogrammmessungen (118) basiert.

3. Verfahren nach Anspruch 2, wobei das Sortieren (206) der Teilmenge (136) der Angaben ferner auf den Konfidenzniveaus basiert, die von der Teilmenge (136) der Angaben angegeben werden.

4. Verfahren nach Anspruch 1, wobei die mehreren Angaben ferner Pulsschlagzählungen (132A, 132B, 132C) des Patienten während der kardialen Ereignisse (126A, 126B, 126C) angeben und wobei das Auswählen (204) der Teilmenge (136) der Angaben ferner auf den Pulsschlagzählungen (132A, 132B, 132C) basiert.

5. Verfahren nach Anspruch 1, wobei das Eliminieren der ersten Angabe darauf basiert, dass eine von der ersten Angabe angegebene Dauer kürzer ist als eine von der zweiten Angabe angegebene Dauer.

6. Verfahren nach Anspruch 1, wobei die kardialen Ereignisse bei dem Patienten (102) am selben Tag stattgefunden haben.

7. Verfahren nach Anspruch 1, wobei das Übermitteln der Angabe das Erzeugen eines Berichts, der die Angabe enthält, umfasst.

8. Verfahren nach Anspruch 1, das ferner das Übermitteln mehrerer Angaben von der sortierten Liste zur medizinischen Überprüfung basierend auf einem Ereignisschwellenwert umfasst.

9. Vorrichtung (112) zum Auswählen, Sortieren und Übermitteln von Angaben zu kardialen Ereignissen, umfassend:
einen Speicher (116); und
einen Hardwareprozessor (114), der kommunikativ mit dem Speicher gekoppelt ist, wobei der Hardwareprozessor ausgelegt ist zum:
Abrufen (202) mehrerer Angaben zu:
kardialen Ereignissen (126A, 126B, 126C) bei einem Patienten (102);
Herzfrequenzen (128A, 128B, 128C) des Patienten (102) während der kardialen Ereignisse; und
Dauern (130A, 130B, 130C) der kardialen Ereignisse;
Auswählen (204) einer Teilmenge der Angaben aus den mehreren Angaben basierend auf den Herzfrequenzen des Patienten während der kardialen Ereignisse und den Dauern der kardialen Ereignisse;
Sortieren (206) der Teilmenge der Angaben basierend auf Herzfrequenzen des Patienten während des kardialen Ereignisses, das von der Teilmenge der Angaben angegeben wird, und basierend auf Dauern der kardialen Ereignisse, die von der Teilmenge der Angaben angegeben werden, um eine sortierte Liste von Angaben zu erstellen; und
Übermitteln (208) einer Angabe von der sortierten Liste, die die erste auf der sortierten Liste ist, zur medizinischen Überprüfung;
wobei das Auswählen (204, 310) der Teilmenge der Angaben das Eliminieren (308) einer ersten Angabe der mehreren Angaben umfasst, als Reaktion auf das Bestimmen (304, 306), dass (i) die erste Angabe einen gleichen Typ von kardialem Ereignis angibt wie eine zweite Angabe der mehreren Angaben und (ii) ein Unterschied zwischen einer von der ersten Angabe angegebenen Herzfrequenz und einer von der zweiten Angabe angegebenen Herzfrequenz geringer ist als ein Unterschiedsschwellenwert.

10. Vorrichtung (112) nach Anspruch 9, wobei die mehreren Angaben basierend auf Elektrokardiogrammmessungen des Patienten erzeugt werden, wobei die mehreren Angaben ferner Konfidenzniveaus der Elektrokardiogrammmessungen angeben und wobei das Auswählen der Teilmenge (136) der Angaben ferner auf den Konfidenzniveaus der Elektrokardiogrammmessungen basiert.

11. Vorrichtung (112) nach Anspruch 10, wobei das Sortieren (206) der Teilmenge (136) der Angaben ferner auf den Konfidenzniveaus basiert, die von der Teilmenge (136) der Angaben angegeben werden.

12. Vorrichtung (112) nach Anspruch 9, wobei die mehreren Angaben ferner Pulsschlagzählungen (132A, 132B, 132C) des Patienten (102) während der kardialen Ereignisse angeben und wobei das Auswählen (204) der Teilmenge (136) der Angaben ferner auf den Pulsschlagzählungen basiert.

13. Vorrichtung (112) nach Anspruch 9, wobei das Eliminieren (308) der ersten Angabe darauf basiert, dass eine von der ersten Angabe angegebene Dauer kürzer ist als eine von der zweiten Angabe angegebene Dauer.

14. Vorrichtung (112) nach Anspruch 9, wobei die kardialen Ereignisse bei dem Patienten (102) am selben Tag stattgefunden haben.

15. Vorrichtung (112) nach Anspruch 9, wobei das Übermitteln der Angabe das Erzeugen eines Berichts, der die Angabe enthält, umfasst.

## Revendications

1. Procédé (200) pour sélectionner, trier et communiquer des indications d'événements cardiaques, comprenant :
l'obtention (202) d'une pluralité d'indications incluant:
des événements cardiaques (126A, 126B, 126C) chez un patient (102) ;
des fréquences cardiaques (128A, 128B, 128C) du patient (102) pendant les événements cardiaques (126A, 126B, 126C) ; et
des durées (130A, 130B, 130C) des événements cardiaques ;
la sélection (204) d'un sous-ensemble (136) d'indications à partir de la pluralité d'indications sur la base des fréquences cardiaques du patient pendant les événements cardiaques et des durées des événements cardiaques ;
le tri (206) du sous-ensemble (136) d'indications sur la base de fréquences cardiaques du patient pendant l'événement cardiaque qui est indiqué par le sous-ensemble (136) d'indications et sur la base de durées des événements cardiaques qui sont indiqués par le sous-ensemble (136) d'indications afin de produire une liste triée d'indications ; et
la communication (208), à partir de la liste triée, d'une indication qui est une première indication dans la liste triée pour un examen clinique ;
dans lequel la sélection (206, 310) du sous-ensemble (136) d'indications comprend l'élimination (308) d'une première indication de la pluralité d'indications en réponse à la détermination (304, 306) du fait que (i) la première indication indique un même type d'événement cardiaque qu'une seconde indication de la pluralité d'indications et du fait que (ii) une différence entre une fréquence cardiaque qui est indiquée par la première indication et une fréquence cardiaque qui est indiquée par la seconde indication est inférieure à un seuil de différence.

2. Procédé selon la revendication 1, dans lequel les indications de la pluralité d'indications sont générées sur la base de mesures d'électrocardiogramme (118) du patient (102), dans lequel les indications de la pluralité d'indications indiquent en outre des niveaux de confiance (134A, 134B, 134C) des mesures d'électrocardiogramme (118) et dans lequel la sélection (204, 310) du sous-ensemble (136) d'indications est en outre basée sur les niveaux de confiance (134A, 134B, 134C) des mesures d'électrocardiogramme (118).

3. Procédé selon la revendication 2, dans lequel le tri (206) du sous-ensemble (136) d'indications est en outre basé sur les niveaux de confiance qui sont indiqués par le sous-ensemble (136) d'indications.

4. Procédé selon la revendication 1, dans lequel les indications de la pluralité d'indications indiquent en outre des comptages de battements (132A, 132B, 132C) du patient pendant les événements cardiaques (126A, 126B, 126C) et dans lequel la sélection (204) du sous-ensemble (136) d'indications est en outre basée sur les comptages de battements (132A, 132B, 132C).

5. Procédé selon la revendication 1, dans lequel l'élimination de la première indication est basée sur le fait qu'une durée qui est indiquée par la première indication est plus courte qu'une durée qui est indiquée par la seconde indication.

6. Procédé selon la revendication 1, dans lequel les événements cardiaques chez le patient (102) se sont produits un même jour.

7. Procédé selon la revendication 1, dans lequel la communication de l'indication comprend la génération d'un rapport qui inclut l'indication.

8. Procédé selon la revendication 1, comprenant en outre la communication d'une pluralité d'indications en provenance de la liste triée pour un examen clinique sur la base d'un seuil d'événement.

9. Appareil (112) pour sélectionner, trier et communiquer des indications d'événements cardiaques, comprenant:
une mémoire (116) ; et
un processeur matériel (114) couplé en termes de communication à la mémoire, le processeur matériel étant configuré pour :
obtenir (202) une pluralité d'indications incluant :
des événements cardiaques (126A, 126B, 126C) chez un patient (102) ;
des fréquences cardiaques (128A, 128B, 128C) du patient (102) pendant les événements cardiaques ; et
des durées (130A, 130B, 130C) des événements cardiaques ;
sélectionner (204) un sous-ensemble d'indications à partir de la pluralité d'indications sur la base des fréquences cardiaques du patient pendant les événements cardiaques et des durées des événements cardiaques ;
trier (206) le sous-ensemble d'indications sur la base de fréquences cardiaques du patient pendant l'événement cardiaque qui est indiqué par le sous-ensemble d'indications et sur la base de durées des événements cardiaques qui sont indiqués par le sous-ensemble d'indications afin de produire une liste triée d'indications ; et
communiquer (208), à partir de la liste triée, une indication qui est une première indication dans la liste triée pour un examen clinique ;
dans lequel la sélection (204, 310) du sous-ensemble d'indications comprend l'élimination (308) d'une première indication de la pluralité d'indications en réponse à la détermination (304, 306) du fait que (i) la première indication indique un même type d'événement cardiaque qu'une seconde indication de la pluralité d'indications et du fait que (ii) une différence entre une fréquence cardiaque qui est indiquée par la première indication et une fréquence cardiaque qui est indiquée par la seconde indication est inférieure à un seuil de différence.

10. Appareil (112) selon la revendication 9, dans lequel les indications de la pluralité d'indications sont générées sur la base de mesures d'électrocardiogramme du patient, dans lequel les indications de la pluralité d'indications indiquent en outre des niveaux de confiance des mesures d'électrocardiogramme et dans lequel la sélection du sous-ensemble (136) d'indications est en outre basée sur les niveaux de confiance des mesures d'électrocardiogramme.

11. Appareil (112) selon la revendication 10, dans lequel le tri (206) du sous-ensemble (136) d'indications est en outre basé sur les niveaux de confiance qui sont indiqués par le sous-ensemble (136) d'indications.

12. Appareil (112) selon la revendication 9, dans lequel les indications de la pluralité d'indications indiquent en outre des comptages de battements (132A, 132B, 132C) du patient (102) pendant les événements cardiaques et dans lequel la sélection (204) du sous-ensemble (136) d'indications est en outre basée sur les comptages de battements.

13. Appareil (112) selon la revendication 9, dans lequel l'élimination (308) de la première indication est basée sur le fait qu'une durée qui est indiquée par la première indication est plus courte qu'une durée qui est indiquée par la seconde indication.

14. Appareil (112) selon la revendication 9, dans lequel les événements cardiaques chez le patient (102) se sont produits un même jour.

15. Appareil (112) selon la revendication 9, dans lequel la communication de l'indication comprend la génération d'un rapport qui inclut l'indication.
